# EUROPEAN PATENT SPECIFICATION

(11) **EP 1 709 191 B1**
(45) Date of publication and mention of the grant of the patent: **25.02.2009**
(21) Application number: 04806773.0
(22) Date of filing: 22.12.2004
(51) Int. Cl.: C12Q 1/68

(54) **OLIGONUCLEOTIDES FOR DETECTION OF LEISHMANIASIS AND METHODS THEREOF**
OLIGONUKLEOTIDE ZUM NACHWEIS VON LEISHMANIASE UND VERFAHREN DAFÜR
OLIGONUCLEOTIDES SERVANT A DETECTER UNE LEISHMANIOSE, ET PROCEDES CORRESPONDANTS

(30) Priority: 23.12.2003 IN DE15982003
(43) Date of publication of application: 11.10.2006
(73) Proprietor: All India Institute of Medical Sciences, Ansari Nagar New Delhi 110029 (IN); Department of Biotechnology (a Department of the Government of India), New Delhi 110 003 (IN)
(72) Inventor: SINGH, Sarman,Dept. of Laboratory Medicine, New Delhi 110029 (IN)
(74) Representative: Winter, Brandl, Fürniss, Hübner Röss, Kaiser, Polte Partnerschaft Patent- und Rechtsanwaltskanzlei
(86) International application number: PCT/IN2004/000395
(87) International publication number: WO 2005/061729

(56) References cited:
- US-A- 5 834 592
- US-A1- 2003 162 182
- BURNS J.M. ET AL: 'Molecular characterization of a kinesin-related antigen of Leishmania chagasi that detects specific antibody in African and American visceral leishmaniasis' PROCEEDINGS OF THE NATIONAL ACADEMY OF SCIENCES OF THE UNITED STATES OF AMERICA vol. 90, no. 2, 15 January 1993, pages 775 - 779, XP002999744

## Description

### TECHNICAL FIELD

The present invention provides a method to detect Leishmaniasis by amplification of the tandem repeat region of Kinesin-related gene from various strains of *L. donavani.* This invention also provides novel primers and an amplification method using these primers to detect and differentiate visceral leishmaniasis (VL) and post kala-azar-dermal leishmaniasis (PKDL) causing strains of *L. donavani.*

### BACKGROUND AND PRIOR ART REFERENCES

Leishmaniasis, a vector-home parasitic disease, is caused by obligate intramacrophage protozoa. It is characterized by diversity and complexity. It presents itself with a wide range of clinical forms. However, there are mainly 4 clinical forms. The Visceral Leishmaniasis (VL), also known as *kala azar,* is the most severe form of the disease, which, if untreated, has a mortality rate of almost 100%. The Cutaneous Leishmaniasis (CL) produces skin ulcers on the exposed parts of the body, such as the face, arms and legs. The number of ulcers may vary from 1 to as many as 200 in some cases, causing serious disability and leaving the patient permanently scarred. The third form is Mucocutaneous Leishmaniasis (MCL), or *espundia.* It can lead to extensive and disfiguring destruction of mucous membranes of the nose, mouth and throat cavities and can involve even the cartilage. The cutaneous form may lead to disseminated form, known as Diffuse Cutaneous Leishmaniasis (DCL). Leishmaniasis is caused by a total of about 21 species, which are transmitted by about 30 species of *Phlebotomine* sand flies [Herwaldt BL., 1999].

Leishmaniasis is presently endemic in 88 countries on five continents: Africa, Asia, Europe, North America and South America and a total of 350 million people are at risk of infection. It is estimated that worldwide 12 million people are affected by leishmaniasis; this figure includes cases with overt disease and those with no apparent symptoms. Of the 1.5-2 million new cases estimated to occur annually, only 600 000 are officially declared. Of the 500 000 new cases of VL, which occur annually, 90%, are in five developing countries: Bangladesh, Brazil, India, Nepal and Sudan. About 90% of all cases of MCL occur in Bolivia, Brazil and Peru and 90% of all cases of CL occur in Afghanistan, Brazil, Iran, Peru, Saudi Arabia and Syria, with 1-1.5 million new cases reported annually worldwide. The geographical distribution of leishmaniasis is limited by the distribution of the sand fly, its susceptibility to cold climates, its tendency to take blood from humans or animals and its capacity to support the internal development of specific species of *Leishmania* [Desjeux P 2001].

In India, VL is a serious problem in Bihar, West Bengal and Eastern Uttar Pradesh where, there is under-reporting of Kala-azar (KA) and post kala-azar dermal leishmaniasis in women and children between 0-9 years of age. The recent epidemics in 1992 of VL killed more than 100,000 people in India and Sudan. Spraying of DDT helped control KA in India, however there are reports of the vector *Phlebotomus argentipes* developing resistance. Also, lymphadenopathy, a major presenting feature in India raises the possibility of a new vector or a variant of the disease [Bora D., 1999].

The post kala-azar dermal leishmaniasis (PKDL) is a sequel to KA in India and Sudan; the disease develops months to years after the patient recovery from KA. Cutaneous lesions characterize the disease and they demonstrate great variability, ranging from hypopigmented macules to erythematous papules and from nodules to plaques. As in leprosy, the wide clinical spectrum of PKDL reflects the immune response of the individual to the *leishmania* organism. Lesions may be numerous and persist for decades. Isolated parasites from the lesions are identical to those causing the original visceral disease.

The clinical and epidemiological findings in leishmaniasis are not pathgnomic and these can mimic several endemic conditions such as malaria, tuberculosis, syphilis and fungal infections. Hence, a laboratory diagnosis is required to confirm the clinical suspicion. The diagnostic tools used for each leishmanial syndrome viz. visceral, cutaneous, and mucocutaneous form vary, but the gold standard in each case remains the demonstration and isolation of the parasite from appropriate tissue [Singh S *et al.,* 2003].

The clinical signs and symptoms are not enough to differentiate VL from other similar conditions such as malaria, tropical splenomegaly syndrome schistosomiasis or cirrhosis with portal hypertension, African trypanosomiasis, milliary tuberculosis, brucellosis, typhoid fever, bacterial endocarditis, histoplasmosis, malnutrition, lymphoma, and leukemia. Hence other diagnostic methods are required [Herwaldt BL, 1999; Davidson RN, 1998]. Amongst these the most specific and standard technique is parasitological demonstration or isolation of the causative agent. Marrow obtained from sternal or iliac crest puncture is a much safer but a painful method. The aspirates are smeared on the glass slide and stained with Romanowsky's stain to demonstrate the amastigote forms of the parasite. However; on culture it can give positive results in up to 80% of the cases. Lymph gland puncture gives positive results in 60% of the cases. Juice is extracted from any enlarged lymph gland and subjected to both direct examination and culture to give the best chance of diagnosis [Williams, J. E, 1995; Manson-Bahr PEC, 1987]. Primary isolation of *L. donovani* is made on solid Novy- MacNeal- Nicolle (NNN) medium having 20-30% rabbit blood or liquid Schneider's insect medium supplemented with 10% v/v foetal calf serum (FCS). Other suitable growth media can also be used particularly for maintaining the subcultures of the promastigotes using FCS or other supplements including human urine [Singh *S et al.,* 2000]. Demonstration of the parasites in the spleen and liver is one of the most accurate methods available to determine leishmanial infections. Ninety percent of the active cases show parasites in splenic and liver aspirates [Williams, J. E, 1995]. Part of the splenic aspirate can be used to make smears for direct microscopic examination and the rest should be cultured. There are several methods for the detection of this disease in patients. The conventional microscopic methods are invasive and painful carrying risk of iatrogenic infections and fatal hemorrhages.

The formol gel test is oldest serological test and has the advantage of being cheap and simple to perform. This test is non-specific since it is based on detecting raised levels of IgG and IgM immunoglobulins [WHO expert committee report, 1991]. Several other tests based on this principle had been in use in past but very rarely used these days. [Singh S, 1999]

There are number of specific serological tests and all have variable sensitivity and specificity for disease diagnosis. Some of these tests include indirect haemagglutination (IHA), counter current immuno electrophoresis (CCIEP), Immuno diffusion (ID) etc. but all these tests are cumbersome and lack sensitivity and specificity and hence not commonly used. Some more commonly used ones are given below:
1. Leishmanin Skin Test (LST) [Singh S, 1999, Sassi A, *et al.,* 1999]
2. Indirect fluorescent antibody test (IFAT) [Williams, J. E, 1995, Gari-Toussaint M, *et al.,* 1994]
3. Direct Agglutination test [Schallig HD *et al.,* 2001]
4. Immunoblotting [Herwaldt BL., 1999; Singh S, 1999; Schallig HD *et al.,* 2001]
5. Antigen Detection [Senaldi G *et* a/., 2001; Attar ZJ *et al.,* 2001]
6. Enzyme linked immunosorbent assay (ELISA) [Martin SK *et al.,* 1998, Rajasekariah GH *et al..* 2001, Schoone GJ *et al.,* 2001]

Raj *et al.* (1999) have developed a recombinant protein r-ORFF of *L. infantum* origin for diagnosis of VL in India. The ORFF protein is encoded in the LD1 locus of chromosome 35 of *L. infantum,* an ELISA with this antigen proved to be sensitive with as little as 5ng of r-ORFF when performed with different groups of patients like confirmed VL, suspected VL, Intermittently treated endemic normal and non-endemic normal. Further the test is in early stage and needs to be evaluated by others and its utility for the field diagnosis is yet to be studied [Raj VS *et al.,* 1999].

Another recombinant antigen, belonging to the kinesin-related gene family of motor proteins, recombinant K39 (rK39) has been shown to be specific for antibodies arising during VL caused by members of the *L. donovani* complex, which include *Leishmania chagasi* and *L. infantum.* This antigen, which is a member of the kinesin-related gene family, encodes a protein with a repetitive epitope, consisting of 39 amino acid residues (K39) is highly sensitive and predictive for onset of acute disease and high antibody titers have been demonstrated in VL patients. [Bums JM Jr *et a*/*.,* 1993; Singh S *et al.,* 1995; Badaro R *et al.,* 1996; Singh S *et al.,* 2002; Maalej IA et al., 2003, US PATENT No 5,411,865; US PATENT No 5,719,263]

### Molecular Methods

Molecular biology is increasingly relevant to the diagnosis and control of infectious diseases. Information on DNA sequences has been extensively exploited for the development of Polymerase chain reaction-based assays for the diagnosis of leishmaniasis and the identification of parasite species. Techniques such as micro arrays and nucleic acid sequence-based amplification will eventually allow rapid screening for specific parasite genotypes and assist in diagnostic and epidemiological studies.

The early diagnosis of leishmaniasis is important in order to avoid severe damage or death of the patient. The routine diagnosis of leishmaniasis relies on either the microscopical demonstration of *Leishmania* amastigotes in aspirates from lymphoid tissue, Liver or Bone marrow aspirates, in slit skin smears or peripheral blood or culturing. However, the retrieval of the sample is uncomfortable to the patient and the isolation of parasite by culturing is time consuming, difficult and expensive. The immunological methods fail to distinguish between past and present infections and are not reliable in the case of immunocompromised patients. Furthermore, none of the serological methods addresses the problem of species identification, which is important for determining appropriate diseases control measures. Patients with cutaneous (CL) or mucocutaneous leishmaniasis (MCL) often have low or no leishmania antibodies, because of the localized character of the disease, and thus serological tests are mostly negative. Molecular approach capable of detecting nucleic acids unique to the parasite in the tissue would address these limitations. Therefore, PCR is an important tool for the diagnosis of CL and MCL. PCR has also been reported very useful for the diagnosis of PKDL. A variety of DNA based detection methods targeting DNA and RNA genes have been developed. PCR has caused a revolution in the diagnosis of Leishmaniasis [Singh S *et al.,* 2003].

Amongst the molecular methods used for clinical diagnosis, PCR has proved to be a highly sensitive and specific technique. A recent study has reported a PCR assay that could detect parasitemia a few weeks before the appearance of any clinical signs or symptoms. Different DNA sequences in the genome of leishmania like ITS region, gp63 locus, telomeric sequences, sequence targets in rRNA genes such as 18s rRNA and SSU-rRNA and both conserved and variable regions in kDNA minicircles are being used by various workers [El Tai NO *etal.,* 2001; Pizzuto M etal.. 2001; Wortman G *etal.* 2001, Monroy Ostria & Sanchez-Tezeda G, 2002, Chiurillo MA *etal.,* 2001]. Using PCR methodology, it is no more essential to undergo invasive methods such as bone marrow, splenic punctures, lymphnode biopsy, liver biopsy etc. or collect large volumes of blood samples. Even a few drops of blood on filter paper may be sufficient. [Da Silva *et al.,* 2004]

In a recent study comparing three different techniques such as PCR fingerprinting, PCR-RFLP and PCR SSCP to reveal the intraspecific polymorphism, the PCR-SSCP technique has been found to be advantageous than the other two for the detection of sequence variation in rRNA genes within the *L. donovani* species. In addition, it can be performed easily and rapidly without prior cultivation of the parasite facilitating detection and identification of the parasite simultaneously [El Tai NO *et al,* 2001]. Another PCR assay assessed by Pizzuto *et al*. (2001), for post therapeutic follow up and the detection of relapses, was found 97% sensitive to peripheral blood and 100% sensitive to bone marrow for detecting leishmania species among HIV-infected patients using SSU rRNA gene target [Pizzuto M *etal.,* 2001]. However there are 2 major disadvantages of SSCP. First, the amounts of mobility differences have little if any correlation to the amount of sequence differences. Thus, the only information that can be gained from SSCP is if PCR amplicons are "identical" or different. Second, the optimal amplicon size for detection of most point mutations is rather small, around 200 bp. The strategies to deal with this limitation (e.g. dideoxy fingerprinting or cutting amplicons with restriction enzymes) are often tedious and do not necessarily give the results desired.

Multiplex PCR in diagnosis and species identification of leishmaniasis: PCR can offer a rapid, sensitive, specific, and low-cost alternative. A number of PCR assays for identification of *Leishmania* at the genus level or for characterization of individual complexes of *L*. *braziliensis, L. mexicana* or *L. donovani* have been described. However, none of these PCR protocols identifies all three complexes in one assay.

Recently in last few years development in the field of molecular biology has led to the development of a simple sensitive and specific one step PCR based assay for differentiating the three complexes of New World *Leishmania.* This method employs different set of primers in a single PCR reaction and known as multiplex PCR. There is a report of use of this method, using the multicopy spliced leader (SL) RNA (mini exon gene) as a target. This assay generates species-specific products of different sizes for L. *braziliensis, L. mexicana,* and *L. donovani* and is suitable for use in non-sophisticated laboratories in countries where leishmaniasis is endemic. In another study *Leishmania* strains were characterized using a single 5' primer and two 3' primers combined in a single multiplex reaction. [Harris *et.al.* 1998, Belli *et.al.* 1998]. Although this method is useful but the chances of non-specific amplification or false positive results are high because of use of multiple primer sets. So, in order to avoid such results, one must select the primers amplifying highly conserved region in the species to avoid non specific amplification.

Several strains might circulate in an endemic area at a given time. Hence, species and strain specific primers have been developed to detect genetic heterogeneity. Recently primers developed by our group could differentiate the Indian strains causing VL and PKDL forms. A multiplex Alu-PCR-like amplification was performed with the cultured L. *donovani* isolates from VL and PKDL patients. The banding pattern of the PCR amplicons could clearly group all the PKDL strains in one group while VL strains had intra-species heterogeneity.

The applicants did extensive search of the patent database with different key words to study the previous work done on the Alu PCR / PCR based diagnosis of leishmaniasis and PCR amplification of the kinesin-related gene to diagnose and differentiate the VL and PKDL causing strains. Discussed below are the few US patents on the subject concerned and the uniqueness of the applicant's invention.

The United States Patent no. 5,411,865 by Reed in May 2, 1995 teaches about the method of detecting anti-leishmania parasite antibodies. The compound disclosed is for a method for detecting anti-Leishmania parasite antibodies to a 230 kDa antigen present in Leishmania chagasi and Leishmania donovani.

The United States Patent no. 5,719,263 by Reed in February 17, 1998 teaches about the 230Kd antigen present in Leishmania species. The compound disclosed is an isolated 230 Kd antigen that is present in Leishmania chagasi and Leishmania donovani, and isolated polypeptides comprising one or a plurality of K39 repeat antigens. Also disclosed are DNAs encoding the 230 Kd antigen and the K39 repeat antigen, and vaccine compositions comprising the antigens.

The above disclosed 230kDa antigen and the isolated polypeptide comprising the K39 repeats are only serological methods and further reported to be not very sensitive in certain geographical areas where VL is highly endemic and caused by *L. donovani.* In contrary, the applicant's invention provides methods and compounds, which deal with molecular diagnostic or Nucleic acid amplification based tests.

The United States Patent no. 5,834,592 by Reed et al., in November 10, 1998 gives information about a an isolated polypeptide comprising an immunogenic portion of a *Leishmania* antigen having the amino acid sequence recited in SEQ ID NO: 4, or a variant of said antigen that differs only in conservative substitutions, modifications or combinations thereof. The antigen is considered to be important in immunodiagnosis and therapy of leishmaniasis. However the applicant invention differs from the compound patented.

The United States Patent no. 5,846,748 by Mandal et al., in December 8, 1998 gives information about method for diagnosing visceral leishmaniasis in a patient by identification of a new key marker namely 9-O-acetylated sialoglycoconjugate. This invention relates to identification of a new key marker namely 9-O-Acetylated sialoglycoconjugate with the help of a known 9-O acetylsialic acid binding lectin, Achatinin-H useful for the diagnosis of visceral leishmaniasis, by a rapid, accurate haemagglutination assay. In contrary, the applicant's invention provides methods and compounds, which deal with molecular diagnostic or Nucleic acid amplification based tests.

The United States Patent no.5, 912,166 by Reed, et al., in June 15, 1999 teaches about compounds and methods for diagnosis of leishmaniasis infection. The compounds provided include polypeptides that contain at least an epitope of the Leishmania chagasi acidic ribosomal antigen LcP0, or a variant thereof. Such compounds are useful in a variety of immunoassays for detecting Leishmania infection and for identifying individuals with asymptomatic infections that are likely to progress to acute visceral leishmaniasis. The polypeptide compounds are further useful in vaccines and pharmaceutical compositions for preventing leishmaniasis. However, the applicant's present invention does not deal with acidic ribosomal antigen LcPO.

The United States Patent No. 6,525,186 by Bebate, et al., in February 2003, is an isolated polynucleotide, comprising a recombinant cDNA encoding a chimeric polypeptide having 4 proteins LiP2a, LiP2b, LiH2a and LiPO of *Leishmania infantum* useful in serological diagnosis of canine leishmaniasis and protein obtained contains at least one antigenic determinant, recognized by serum from dogs with Visceral Leishmaniasis. In contrary, the applicant's invention provides methods and compounds, which deal with molecular diagnostic or Nucleic acid amplification based tests.

The United States Patent No. 6,613,337 by Reed, et al., in September 2, 2003, deals with a fusion protein and a physiologically acceptable carrier, wherein the fusion protein comprises the amino acid sequence of SEQ ID NO: 24, for use in the therapy and diagnosis of leishmaniasis. The combination contains polypeptides that comprise immunogenic portions of M15, Ldp23, Lbhsp83, Lt-1 and LbelF4A. However, the applicant's present invention does not deal with application of fusion protein.

The United States Patent No. 6,638,517 by Reed, et al., in October 28, 2003, Leishmania antigens for use in the therapy and diagnosis of leishmaniasis teaches compositions and methods for preventing, treating and detecting leishmaniasis and stimulating immune responses in patients. The compounds provided include polypeptides that contain an immunogenic portion of one or more Leishmania antigens, or a variant thereof. The patent also discloses vaccines and pharmaceutical compositions comprising such polypeptides, or polynucleotides encoding such polypeptides, are also provided and may be used, for example, for the prevention and therapy of leishmaniasis, as well as for the detection of Leishmania infection.

United States Patent Application 20030162182, Salotra Poonam et al., August 28, 2003, Species-specific PCR assay for detection of Leishmania donovani in clinical samples of kala-azar and post kala-azar dermal leishmaniasis teaches methods and compounds for the polymerase chain reaction (PCR) assay for the diagnosis of leishmaniasis using specific novel oligonucleotide primers for the identification of *Leishmania donovani* parasites in clinical samples.

The applicant's invention uses a target in the genomic DNA of *leishmania* that is entirely different from the Salotra *et al.* (Us application No. 20030162182), work, where they amplify the minicircles in the kinetoplast DNA which is a type of mitochondrial DNA and very les in quantity, thereby providing less primer targets yielding to poor sensitivity. The present invention uses genomic DNA as a target for the PCR amplification which is in abundance in the parasite and thus better sensitvity. The DNA sequences amplified are from genomic DNA whereas Dr. Salotra method amplifies a region of the mitochondrial DNA, which is difficult to isolate as compared to genomic DNA and requires more expertise and facilities. Also the amount of mitochondrial DNA (K-DNA) isolated is much lower than the amount of genomic DNA isolated. The method developed by Salotra cannot differentiate between VL and PKDL forms of leishmania. So the present invention is more convenient to perform with having ability of differentiating the species more specifically.
The present invention provides a direct method of detecting Leishmania by amplification of the conserved repeat region of the kinesin-related gene, whereas all the other reported methods are based on the polypeptide derived from the kinesin-related gene (antigen-antibody tests). Another feature of this invention is that, the PCR method can differentiate between visceral Leishmaniasis (VL) and post kala-azar dermal leishmaniasis (PKDL).

### OBJECTS OF THE INVENTION

The main object of the present invention is to develop novel oligonucleotide primers for amplification of the kinesin-related gene of *Leishmania* species. Further the object is to design primers based on the repetitive region of the kinesin-related gene for PCR amplification.

Another object of the invention is to develop a method for PCR amplification to detect Leishmaniasis in the patients infected with *Leishmania donovani* strains based on the conserved repeat region of kinesin-related gene of *Leishmania* species using novel oligonucleotide primers.

Another object of the present invention is to develop a method for detection and differentiating VL and PKDL causing strains of leishmaniasis using the novel oligonucleotide primers.

Yet another object of the present invention is for a method of detection for leishmaniasis from a sample which is selected from either clinical samples or culture samples.

Further object of the present invention is to develop a diagnostic kit for detecting and differentiating the VL /PKDL strains/forms of the leishmaniasis, comprising of novel oligonucleotide primers, a reaction buffer, DNA polymerase, *Taq* polymerase.

### BRIEF DESCRIPTION OF ACCOMPANYING DRAWING

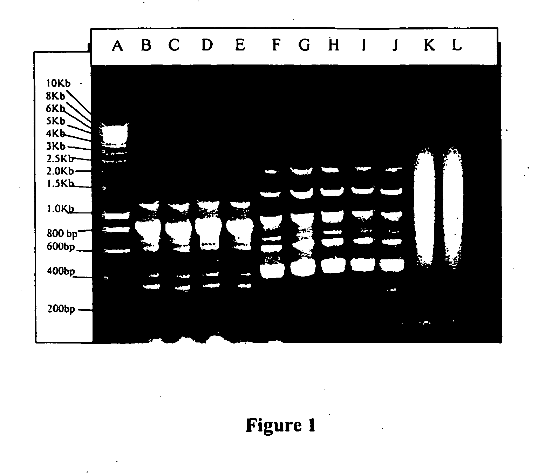

- **Figure 1:**: PCR amplification of the DNA from various VL and PKDL causing strains of *Leishmania donovani.* The lanes depict the following: Lane A represents molecular weight marker, PKDL (Lane B, strain RMP-240; Lane C, strain RMP-142; Lane D, strain RMP-155; Lane E, strain RMP-19); and *Leishmania donovani* strains causing visceral diseases [Lane F, DD-8 (WHO reference strain); Lane G, strain RMR-1; Lane H, strain KE-16, Lane I, UR-6]; Lane J is a Clinical (Blood) sample of patient positive for visceral Leishmaniasis, Lane K, Healthy Human DNA Sample and Lane L, is a blood sample from a patient with CMV infection (Disease Control).

### SUMMARY OF THE INVENTION

Accordingly, the present invention provides novel oligonucleotide primers for amplification of the kinesin-related gene of *Leishmania* species selected from SEQ ID NO: 1, SEQ ID No: 2, SEQ ID NO: 3 and SEQ ID NO: 4, wherein the novel oligonucleotide primers are designed based on the tandem repeat region of the kinesin-related gene of *Leishmania* species.

The present invention also provides a method based on the amplification of the tandem repeat region of Kinesin-related gene from various strains of *L. donavani* using the primers (SEQ ID NO: 1, SEQ ID No: 2, SEQ ID NO: 3 and SEQ ID NO: 4) to detect and differentiate visceral leishmaniasis (VL) and post kala-azar-dermal leishmaniasis (PKDL) causing strains of leishmaniasis. The invention provides a method using multiplex PCR for detecting and differentiating visceral leishmaniasis (VL) and post kala-azar-dermal leishmaniasis (PKDL) causing strains of *Leishmania donovani* in a sample, comprising isolating DNA from a sample; amplifying the target region from the DNA using novel oligonucleotide primers and heat stable DNA polymerase to obtain amplified fragments; separating the amplified fragments and analyzing the fragments to detect and differentiate *VL* and *PKDL* causing strains of *Leishmania donovani* based on the banding pattern of the amplified fragments. In addition, the present invention provides a diagnostic kit for detection and differentiation of VL and PKDL causing strains of the *Leishmania donovani.*

### DETAILED DESCRIPTION OF THE INVENTION

In accordance, the present invention provides novel oligonucleotide primers for amplification of the kinesin-related gene of *Leishmania* species selected from SEQ ID NO: 1, SEQ ID No: 2, SEQ ID NO: 3 and SEQ ID NO: 4.

An embodiment of the present invention provides for a method for detecting and differentiating visceral leishmaniasis (VL) and post kala-azar-dermal leishmaniasis (PKDL) causing strains of *Leishmania donovani,* the said method comprising the steps of:
*a)* isolating DNA from sample;
*b)* amplifying the target region from the DNA of step (a) using novel oligonucleotide primers having SEQ ID NO: 1, SEQ ID No: 2, SEQ ID NO: 3 and SEQ ID NO: 4 and heat stable DNA polymerase to obtain amplified fragments;
*c)* separating the amplified fragments of step (b); and
*d)* analyzing the fragments of step (c) to detect and differentiate *VL* and *PKDL* causing strains of *Leishmania donovani* based on the banding pattern of the amplified products.

In another embodiment of the present invention provides for a method wherein the sample for detection is selected from either clinical samples or culture samples.

Yet another embodiment of the present invention provides for a method wherein the sample for detection is selected from a group consisting of blood, bone marrow aspirate, bone marrow biopsy, splenic aspirate, splenic biopsy, liver aspirate, liver biopsy, lymph node aspirate, lymph node biopsy, skin scrapping, slit biopsy and other tissue materials.

Still another embodiment of the present invention provides for a method wherein the use of heat stable DNA polymerase preferably *Taq* polymerase and the amplification is carried out by polymerase chain reaction.

In another embodiment of the present invention provides for a method wherein separation of the amplified products is by gel electrophoresis and the detection of the amplified products is by ethidium bromide or other DNA stains.

Further embodiment of the present invention provides for a kit for detection and differentiation of VL and PKDL causing strains of the *Leishmania donovani,* comprising oligonucleotide primers having SEQ ID NO: 1, SEQ ID No: 2, SEQ ID NO: 3, SEQ ID NO: 4, reaction buffer, *Taq* polymerase, DNA marker, a positive control sample, a negative control sample and instruction manual.

The present invention relates a method for detection of *Leishmaniasis* wherein the protozoan parasites of the genus *Leishmania* are the causative agents of visceral leishmaniasis (VL), also called kala-azar (KA). KA is a symptomatic infection of the liver, spleen and bone marrow caused by organisms of *Leishmania donovani* complex. PKDL (Post kala-azar dermal leishmaniasis) is an unusual dermatosis that develops as a sequel of KA, producing gross cutaneous lesions in the form of hypopigmented macules, erythema and nodules. The disease is relatively common in the Indian subcontinent and less frequent in East Africa, but exceptional in the American and European continents. Detection and characterization of *Leishmania* from patients of both KA and PKDL is important for deciding treatment regimens as well as for understanding the disease epidemiology. In many patients the dermal manifestations are seen even when the patient never had visceral form hence the term post-kala-azar dermal leishmaniasis is a misnomer. It is also seen that no kala-azar patient has ever developed PKDL once he/she has migrated to a PKDL non-endemic area after kala-azar treatment. Therefore, the applicant proposed a hypothesis that VL and PKDL causing strains of *Leishmania donovani* are different. To elucidate the proposed hypothesis, the applicant successfully designed and standardized an Alu-PCR and its primers to differentiate between these two strains.

The present invention provides a unique PCR amplification method to amplify the Kinesin-related gene of different Indian isolates of *Leishmania donovani.* This has been developed by the applicant to analyze genetic differences of the strains causing VL and PKDL on the basis of number and size of the bands as a result of PCR assay. The PCR assay for detection and differentiation between the strains of *Leishmania donovani* that cause visceral leishmaniasis and strains that cause post kala-azar-dermal leishmaniasis was developed using the following sets of PCR primers:
Forward Primer (SS-KIN 1): SEQ ID NO: 1
   5' CTAGAGCAGCAGCTTCG 3' (17 oligomer)
Forward Primer (SS-KIN 3): SEQ ID NO: 2
   5' CTTGAGCAGCAGCTTCG 3' (17 oligomer)
Reverse Primer (SS-KIN 2): SEQ ID NO: 3
   5' CGTGGCCCTCGTGTTCT 3' (17 oligomer)
Reverse Primer (SS-KIN 4) SEQ ID NO: 4
   5' CGCGGCCCTCGTGTCCT 3' (17 oligomer)

The invention further provides a method to differentiate VL and PKDL strains using an the above primers sets having SEQ ID NO: 1, SEQ ID NO: 2, SEQ ID NO: 3 and SEQ I D NO: 4, which are designed on the basis of consensus, repetitive 117bp sequences in the Kinesin-related gene of *Leishmania donovani* strain MHOM/IN/DD8. The present invention teaches improved methods for differentiation of VL and PKDL causing strains of Leishmania *donovani* based on the PCR amplification of the Kinesin-related gene.

The present invention is described below. Although methods similar or equivalent to those described herein can be used in the practice or testing of the present invention, the preferred methods and materials are described below. The materials, methods, and examples are illustrative only and are not intended to be limiting. Other features and advantages of the invention will be apparent from the detailed description, and from the claims.

### GROWTH OF STRAINS

Parasites were initially isolated as Promastigotes in Novy-Mac Neal Nicolle (NNN) [Sundar, et al., 2002] medium from clinical samples of Kala-azar and post Kala-azar dermal leishmaniasis patients and subsequently adapted to grow at 25°C in Medium 199 containing 10% heat inactivated FCS. For routine maintenance, samples of the inoculum containing parasites were introduced aseptically into culture tubes with 4ml of Medium-199 [Sundar, et al., 2002] supplemented with 10% FCS. The tubes were placed in cooled incubator at 25°C and the growth was monitored at regular intervals by microscopy. For mass cultivation of the parasite, samples of inoculum containing parasites were introduced aseptically into 200ml of M 199 containing 10%FCS in a 500ml tissue culture flask and incubated in a cooled incubator at 25°C until mid log phase (7-10 days). The parasites were then harvested and used for nuclear DNA isolation.

### STRAINS- CULTURE MAINTAINENCE

The VL and PKDL strains, as described herein were isolated from various parts of India (Table 1) and maintained in Medium 199 supplemented with 10% fetal calf serum and mass culture propagation for DNA isolation for PCR were done in medium 199 with 5% FCS +5% human urine (post menopausal female) and culture flasks incubated with agitation at 17-20°C in a BOD incubator

**Table1:**

| Strain ID | Source | Geog. Location | Disease |
|---|---|---|---|
| 1. HM/IN/DD8 | WHO std. strain | Bihar | VL |
| 2. HM/IN/UR6, | IICB, Calcutta | West Bengal | VL |
| 3. HM/IN/Ag83, | IICB, Calcutta | West Bengal | VL |
| 4. HM/IN/SS, | PGIMER, Chd. | Bihar | VL |
| 5. HM/IN/LD183, | Our Lab, AIIMS | Bihar | VL |
| 6. HM/IN/KE16, | Our Lab, AIIMS | Bihar | VL |
| 7. HM/IN/J1, | Our Lab, AIIMS | Bihar | VL |
| 8. HM/IN/J2, | Our Lab, AIIMS | Bihar | VL |
| 9. HM/IN/J3, | Our Lab, AIIMS | Bihar | VL |
| 10. HM/IN/RMRI, | RMRI, Patna | Bihar | PKDL |
| 11. HM/IN/RMP7, | RMRI, Patna | Bihar | PKDL |
| 12. HM/IN/RMP8, | RMRI, Patna | Bihar | PKDL |
| 13. HM/IN/RMP142, | RMRI, Patna | Bihar | PKDL |
| 14. HM/IN/RMP155, | RMRI, Patna | Bihar | PKDL |
| 15. HM/IN/RMP240, | RMRI, Patna | Bihar | PKDL |
| 16. HM/IN/RS, | IICB, Calcutta | Not known | VL |
| 17. HM/IN/MF, | IICB, Calcutta | Not known | VL |
| 18. HM/IN/GEI, | IICB, Calcutta | Not known | VL |
| 19. HM/IN/GEIV | IICB, Calcutta | Not known | VL |

### DNA ISOLATION

The parasites in their mid log phase was harvested by centrifuging at 5000 rpm in a refrigerated centrifuge. Parasite nuclear DNA was isolated following standard protocol [Lu H.G. *et al.,* 1994] with minor modifications. Approximately 1-5 X 10⁹ promastigotes were lysed in 10 volumes of lysis buffer (NaCl, 100 mM, Tris-HCl, 10mM (pH 8.0), EDTA 10mM, Proteinase K/ml 100µg, Sarcosyl 1.5%) at 60 °C for 3 hours. The kinetoplast DNA networks were sedimented by centrifugation at 27,000 X g for I hour and resuspended in TE buffer (Tris-HCl (pH 8.0) 10mM, EDTA (pH 8.0) 1mM). The nuclear DNA was isolated from the supernatants left after sedimentation of the kinetoplast DNA. These supernatants were incubated overnight for further digestion of proteins at 65° C. The nuclear DNA was subjected to several cycles of phenol/chloroform extractions by adding equal volume of phenol/chloroform mixture, mixing thoroughly followed by sedimentation by centrifugation at 5000 rpm for 15 minutes. The nuclear DNA was precipitated by adding 1/10^{th} the volume of 3M-sodium acetate and 2 volumes of 100% ethanol mixed well and incubated at -20°C for 1 hour. The mixture was sedimented by centrifugation at 5000 rpm for 30 minutes at 4°C. The pellet was washed with 70% ethanol, dried and resuspended in TE buffer. The concentration and purity of the DNA was measured by taking OD at 260/280nm. The DNA was also checked using agarose gel electrophoresis using standard DNA as marker for quantification. The DNA was stored at -70°C until use.

The DNA from clinical samples is extracted by adding 300µl of patient whole blood to RBC or tissue lysis solution in 1.5ml Microfuge tube followed by mixing and incubation at room temperature for 30 minutes and treatment given with genomic DNA lysis solution, and centrifuged at 12,000 rpm for 5 minutes, carefully removed all but 30-50 µl of supernatant and to the supernatant add 200 µl of Instagene Matrix (Bio-Rad, USA) to the tube, after incubation at 56°C for 30 minutes, the contents vortexed at high speed for 10 seconds, heated at 100° C in a heating block for 5 minutes, vortexed again and finally re-centrifuged at 12,000 rpm for 5 minutes, 20µl of the isolated DNA from the supernatant was taken for PCR.

### PCR Assay

The PCR assay was standardized using the DNA isolated from the cultures of VL and PKDL causing strains of *Leishmania Donovan.* These cultures were maintained under laboratory conditions using conventional methods. The PCR assay was further carried out using the whole blood sample of *Leishmania donovani* patients for standardization. The PCR amplification was carried out using all the four novel oligonucleotide primers namely SEQ ID NO: 1, SEQ ID NO: 2, SEQ ID NO: 3 and SEQ ID NO: 4. The details of the primers are given below.
Forward Primer (SS-KIN 1): SEQ ID NO: 1
5' CTAGAGCAGCAGCTTCG 3' (17 oligomer)
Forward Primer (SS-KIN 3): SEQ ID NO: 2
5' CTTGAGCAGCAGCTTCG 3' (17 oligomer)
Reverse Primer (SS-KIN 2): SEQ ID NO: 3
5' CGTGGCCCTCGTGTTCT 3' (17 oligomer)
Reverse Primer (SS-KIN 4) SEQ ID NO: 4
5' CGCGGCCCTCGTGTCCT 3' (17 oligomer)

The assay can be termed as multiplex PCR assay. The novel primers were designed based on the consensus repetitive 117 bp region of the Kinesin-related gene of *Leishmania donovani.* The PCR amplification method of the present invention was based on the data concerning structure and organization of repetitive elements in the human genome and having similarity to the sequences in the *Leishmania* species and hence is called as Alu-PCR [Piarrous R *et al.,* 1993]. It has been observed that the primers originating from repetitive sequences recognize and differentiate the locus in a specific manner on the basis of the size of the repetitive element present in the species, which varies in their sizes due to the intron sequences (non-coding regions).

The novel oligonucleotide primers having SEQ ID NO: 1, SEQ ID NO: 2, SEQ ID NO: 3 and SEQ ID NO: 4 were designed based on the consensus, repetitive 117 bp sequence of the Kinesin-related gene of *Leishmania donovani* strain MHOM/IN/DD8. All the four primers (SEQ ID NO: 1, SEQ ID NO: 2, SEQ ID NO: 3 and SEQ ID NO: 4) were employed in the PCR assay for the detection of *Leishmaniasis* using multiplex PCR strategy.

PCR amplification of the kinesin-related gene of different Indian isolates of L. *Donovani* and clinical samples positive for *Leishmaniasis* (serologically positive) were carried out following the method exclusively developed by the applicant using the novel primers having SEQ ID NO: 1, SEQ ID NO: 2, SEQ ID NO: 3 and SEQ ID NO: 4. These primers were uniquely designed based on the 117bp fragment of the kinesin-related gene to amplify the repeated region of the kinesin-related gene. The PCR amplified products were electrophoresed in 2.0% to 2.5% agarose gel and stained with ethidium bromide. The products were visualized under an UV-transilluminator (UVP) for identification of banding pattern.

The present invention teaches a method for detection of VL and PKDL causing strains of *Leishmania donovani* based on novel oligonucleotide primers designed for amplification of the kinesin-related gene. This method is based on multiplex PCR amplification and employs four primers (SEQ ID NO: 1, SEQ ID NO: 2, SEQ ID NO: 3 and SEQ ID NO: 4) in a single reaction mixture. The PCR amplified products were resolved on gel electrophoresis and detected by standard methods. The banding patterns from the various samples were analyzed for detection of VL and PKDL causing strains of *Leishmania donovani.*

### The detailed procedure of the PCR assay is given below:

The PCR assay was carried out in 50 µl reaction mixture for the various samples for detection of VL and PKDL causing strains of *Leishmania donovani.*

Each 50 µl reaction mixture contain 100 ng of nuclear DNA (isolated from samples), 200 uM each of deoxyribonucleotide triphosphates (dNTPs), 1.5 units of Taq DNA Polymerase, 5 ul of 10 X PCR buffer (100 mM TAPS (pH 8.8), 15 mM MgCl2, 500 mM KCI and 0.1% gelatin). The reaction mixture was incubated at temperature of 94°C for 5 min before starting the PCR amplification cycles. The temperatures used for amplification cycles were 94°C for 60s, 52°C for 60s, 72°C for 60s. This was carried out for 25-36 cycles followed by 72°C for 10 min for extension. The PCR amplified products were electrophoresed in 2.0% to 2.5% agarose gels, stained with ethidium bromide and visualized on an UV-transilluminator to detect the banding pattern of the products from the various samples assayed.

The banding pattern of the amplified DNA products was different for VL and PKDL causing strains as observed in Figure 1. A ladder banding pattern was obtained for both the VL and PKDL strains however the banding pattern was different for both these strains. The number of amplified products varied from 8-10 for VL causing strains whereas it was 6-8 bands in PKDL causing strains as seen in Figure 1.

All the four primers were used for amplification at equimolar concentration in a single PCR reaction mixture. It has been observed that primers originating from repetitive sequences recognize a locus in a specific manner likewise the primers SEQ ID NO: 1, SEQ ID NO: 2, SEQ ID NO: 3 and SEQ ID NO: 4 bind in strain specific manner. The amplified products showed distinct ladder pattern of bands for VL and PKDL causing strains, in which the number of bands for VL strains were 8-10 whereas for PKDL strains 6-8 bands were detected. Most of the PCR amplified products were in the size range of 0.2-2.0 kb after PCR amplification from VL strains while in the case of PKDL strains the amplified products were in the range of 0.2 to 1.2 kb in length.

The ladder of amplified products showed distinct pattern of banding for VL strains and PKDL strains, in which the number of bands for VL strains were 8-10 and for PKDL strains 6-8. A representative banding pattern of PCR amplified products is depicted in Figure 1. The figure 1 clearly shows differences in banding pattern between the PKDL and VL causing strains. Based on the banding pattern of the amplified products the *Leishmania* strains from kala-azar (visceral leishmaniasis, VL) patients showed more bands as compared to bands amplified from PKDL causing strains. Bands were amplified in the range 0.2 to 2.0 kb in VL strains which are namely of size 2.0kb, 1.4kb, 1.0kb, 0.7kb, 0.6kb, 0.45 kb and 0.4kb. Faint bands were also detected at around 0.2kb. An intense signal was observed in all these lanes (VL) around 0.45-0.4 kb. This may be due to the presence of doublet bands. This prominent band of 0.45 kb size was absent from PKDL strains (Fig 1).

In the case of PKDL (dermal leishmaniasis of Bihar) causing strains, the number of PCR bands was significantly less. The number was between 6-8 bands. All the PCR amplified products (seen as bands in the fig 1) were observed in the range of 0.20-1.2 kb. Among this the most prominent were bands of sizes 1.2Kb, 0.85Kb, 0.8K, 0.6 KB, 0.4 KB and 0.36 bands. A faint band at around 0.2Kb was observed in PKDL strains (Fig 1). The band at 0.85 showed intense signal and may be a doublet of two bands of sizes 0.85 and 0.8 Kb. Significantly this prominent band was absent in VL strains. In addition, negative controls were included in the PCR assay using the same primers and PCR conditions. These are represented in Figure 1( see lanes K and L). The controls are healthy human DNA sample ( Lane K ) and human DNA sample infected with CMV (Lane L).Av smear was obtained as excess DNA amount ( template ) was taken for the analysis.

After disclosing the primers to differentiate the two causative strains of *Leishmania,* the applicant found that dermal manifestations of *Leishmaniasis* in Bihar and adjoining areas are due to *in-vivo* hybridization and development of quasi species. This invention will help in identifying the organism and its associated disease, whether it will cause VL and PKDL form when the source of isolation is not known. This invention will also help in identifying the specific strain and to trace the source of infection (reservoir) the issue, which has remained unresolved in India till date.

These results, also suggests that VL and PKDL causing agents are genetically different. The following factors need consideration namely.
1.) PKDL being considered to be the sequel of infection with *Leishmania donovani*
2.) The presence of kinesin-related gene conserved only in visceralising species but minor genotypic differences between VL and PKDL isolates imply that, PKDL may be due to recombination between the two *Leishmanial species* co-infecting the same host and then evolving a new strain causing PKDL.

The use of multiplex PCR using SEQ ID NO: 1, SEQ ID NO: 2, SEQ ID NO: 3 and SEQ ID NO: 4 primers is for detection of *Leishmania donovani* infection and to differentiate the strains whether these will cause visceral form of Leishmaniasis (kala-azar) or a dermal form of Leishmaniasis in Bihar commonly known as PKDL.

The invention is further illustrated with the following examples and these examples are not to limit the scope of the invention.

### EXAMPLES

### Example 1

**Growth of Strains:** Parasites were initially isolated as Promastigotes in NNN medium from clinical samples of Kala-azar and post Kala-azar dermal leishmaniasis patients and subsequently adapted to grow at 25°C in Medium 199 containing 10% heat inactivated FCS. For routine maintenance, samples of the inoculum containing parasites were introduced aseptically into culture tubes with 4ml of medium 199 supplemented with 10% FCS. The tubes were placed in cooled incubator at 25°C and the growth was monitored at regular intervals by microscopy. For mass cultivation of the parasite, samples of inoculum containing parasites were introduced aseptically into 200ml of M199 containing 10%FCS in a 500ml tissue culture flask and incubated in a cooled incubator at 25°C until mid log phase (7-10 days). The parasites were then harvested and used for nuclear DNA isolation

### Example 2:

**DNA Isolation**: The parasites in their mid log phase was harvested by centrifuging at 5000 rpm in a refrigerated centrifuge. Parasite nuclear DNA was isolated following standard protocol [Lu H.G. *et al*., 1994] with minor modifications. Approximately 1-5 X 10⁹ promastigotes were lysed in 10 volumes of lysis buffer (NaCl, 100 mM, Tris-HCl, 10mM (pH 8.0), EDTA 10mM, Proteinase K/ml 100µg, Sarcosyl 1.5%) at 60° C for 3 hours. The kinetoplast DNA networks were sedimented by centrifugation at 27,000 X g for I hour and resuspended in TE buffer (Tris-HCl (pH 8.0) 10mM, EDTA (pH 8.0) 1mM). The nuclear DNA was isolated from the supernatants left after sedimentation of the kinetoplast DNA. These supernatants were incubated overnight for further digestion of proteins at 65° C. The nuclear DNA was subjected to several cycles of phenol/chloroform extractions by adding equal volume of phenol/chloroform mixture, mixing thoroughly followed by sedimentation by centrifugation at 5000 rpm for 15 minutes. The nuclear DNA was precipitated by adding 1/10^{th} the volume of 3M-sodium acetate and 2 volumes of 100% ethanol mixed well and incubated at -20°C for I hour. The mixture was sedimented by centrifugation at 5000 rpm for 30 minutes at 4°C. The pellet was washed with 70% ethanol, dried and resuspended in TE buffer. The concentration and purity of the DNA was measured by taking OD at 260/280nm. The DNA was stored at -70°C until use.

### Example 3

### PCR assay for Leishmaniasis:

The PCR assay was carried out using the DNA isolated ( Example 2 gives details for DNA isolation ) from the various strains as given in Table 1. The PCR amplification was carried out using all the four novel oligonucleotide primers namely SEQ ID NO: 1, SEQ ID NO: 2, SEQ ID NO: 3 and SEQ ID NO: 4.. The novel primers were designed based on the consensus repetitive 117 bp region or the Kinesin-related gene of *Leishmania donovani.* The PCR assay can also be termed as multiplex PCR. The details of the primers are given below.
Forward Primer (SS-KIN 1): SEQ ID NO: 1
5' CTAGAGCAGCAGCTTCG 3' (17 oligomer)
Forward Primer (SS-KIN 3): SEQ ID NO: 2
5' CTTGAGCAGCAGCTTCG 3' (17 oligomer)
Reverse Primer (SS-KIN 2): SEQ ID NO: 3
5' CGTGGCCCTCGTGTTCT 3' (17 oligomer)
Reverse Primer (SS-KIN 4) SEQ ID NO: 4
5' CGCGGCCCTCGTGTCCT 3' (17 oligomer)

Each 50 µl reaction mixture contains 100 ng of nuclear DNA (isolated from samples), 200 uM each of deoxyribonucleotide triphosphates (dNTPs), 1.5 units of Taq DNA Polymerase, 5 ul of 10 X PCR buffer (100 mM TAPS (pH 8.8), 15 mM MgCl2, 500 mM KCI and 0.1 % gelatin). The reaction mixture was incubated at temperature of 94°C for 5 min before starting the PCR amplification cycles. The temperatures used for amplification cycles were 94°C for 60s, 52°C for 60s and 72°C for 60s. This was carried out for 35-36 cycles followed by 72°C for 10 min for extension. The PCR amplified products were electrophoresed in 2%. to 2.5% agarose gels, stained with Ethidium Bromide and visualized on an UV-transilluminator to detect the banding pattern of the products from the various samples assayed.

All the four primers were used for amplification at equimolar concentration in a single multiplex -PCR reaction mixture. It has been observed that under certain conditions, primers originating from repetitive sequences recognize a locus in a specific manner; likewise the primers SEQ ID NO: 1, SEQ ID NO: 2, SEQ ID NO: 3 and SEQ ID NO: 4 bind in strain specific manner. The ladder of amplified products showed distinct pattern of bands for VL strains and PKDL strains, in which the number of bands for VL strains were 8-10 and for PKDL strains 6-8 bands were detected. Most of the PCR amplified products were in the size range of 0.2-2.0 kb from VL strains while in PKDL strains the bands ranged from 0.20 1.2 kb. These are shown in Figure1 and the details are given in the description above. Based on the banding pattern after amplification, it is clear that VL and PKDL causing strains of *Leishmania donovani* can be differentiated. From Fig.1, it is clear that 2 bands of molecular weight 1.4 Kb and 2.0 Kb are amplified in samples which are VL causing strains but are absent in PKDL causing strains of *Leishmania donovani.* An other important differencd observed in Fig. 1 is the presence of a prominent band with strong signal in VL strains around 0.45 Kb , which is absent in PKDL strains. Simiarly, an prominent band with strong signal around 0.8 Kb is present in PKDL strains which is absent in VL strains. These differences in banding pattern can be used to differentiate between VL and PKDL causing strains of *Leishmania donovani.*

### Example 4

### Direct PCR Assay:

The DNA from clinical samples is extracted by adding 25µl of patient whole blood to 1 ml of sterile distilled water in 1.5ml Microfuge tube followed by mixing and incubation at room temperature for 30 minutes, centrifuged at 12,000 rpm for 5 minutes, carefully removed all but 30-50 µl of supernatant and added 200 µl of Instagene Matrix (Bio-Rad, USA) to the tube, after incubation at 56°C for 30 minutes, the contents vortexed at high speed for 10 seconds, heated at 100° C in a heating block for 5 minutes, vortexed again and finally re-centrifuged at 12,000 rpm for 5 minutes, 20µl of the isolated DNA from the supernatant was taken for PCR. Further, in the experiment 100-150 ng of nuclear DNA from different isolates were amplified for 36 cycles in 50 µl reaction mixtures, each containing. 200 µM each of deoxynucleoside triphosphates (dNTPs), 1.5 units of *Taq DNA Polymerase* (Perkin Elmer) and 5 µl of 10 X PCR buffer (100 mM TAPS (pH 8.8), 15 mM MgCl₂, 500 mM KCI and 0.1% gelatin). The working concentration of each primer was 0.5 µM. The temperature cycles used were: 94°C for 10 min; 94°C for 60s, 52°C for 60s, 72 °C for 60s followed by 72 °C for 10 min. The PCR products were electrophoresed in 1.5%-2.5% agarose gels, stained with Ethidium Bromide and visualised on an UV-transilluminator. The data obtained from this assay was similar to the data obtained as shown in Example 3. The amplified products showed distinct ladder pattern of bands for VL and PKDL causing strains, in which the number of bands for VL strains were 8-10 whereas for PKDL strains 6-8 were detected. Most of the PCR amplified products were in the size range of 0.2-2.0 kb after PCR amplification from VL strains while in the case of PKDL strains the amplified products were in the range of 0.2 to 1.2 kb in length.

### REFERENCES:

1. Attar ZJ, Chance ML, el-Safi S, Carney J, Azazy A, El-Hadi M, Dourado C, Hommel M. 2001. Latex agglutination test for the detection of urinary antigens in visceral leishmaniasis. Acta Trop.78 (1): 11-6.
2. Badaro, R., D. Benson. M. C. Eulalio. M. Freire, S. Cunha, E. M. Netto, D. Pedral-Sampaio, C. Madureira. J. M. Burns, R. L. Houghton, J. R. David, and S. G. Reed. 1996. r K39: a cloned antigen for Leishmania chagasi that predicts active visceral leishmaniasis. J. Infect. Dis. 173:758-761.
3. Belli A, Rodriguez B, Aviles H and Harris E. Simplified Polymerase Chain Reaction detection of new world Leishmania in clinical specimen of cutaneous leishmaniasis. Am. J. Trop. Med. Hyg. 58(1), 102-109 (1998).
4. Bora D. 1999. Epidemiology of visceral leishmaniasis in India. Natl Med J India. 12(2): 62-8.
5. Burns JM Jr, Shreffler WG, Benson DR, Ghalib HW, Badaro R, Reed SG. 1993. Molecular characterization of a kinesin-related gene antigen of Leishmania chagasi that detects specific antibody in African and American visceral leishmaniasis. Proc Natl Acad Sci, U S A. 90(2): 775-9.
6. Chiurillo MA, Sachdeva M, Dole VS, Yepes Y, Miliani E, Vazquez L. 2001. Detection of Leishmania causing visceral leishmaniasis in the old and new worlds by a polymerase chain reaction assay based on telomeric sequences. Am. J. Trop. Med.Hyg. 65 (5), 573-82.
7. Davidson RN. 1998. Practical guide for the treatment of leishmaniasis. Drugs. 56(6): 1009-18.
8. Desjeux P. 2001. The increase in risk factors for leishmaniasis worldwide. Trans R Soc Trop Med Hyg. 95(3): 239-43.
9. Da Silva ES, Gontijo CM, Pacheco Rda S, Brazil RP Diagnosis of human visceral leishmaniasis by PCR using blood samples spotted on filter paper. Genet. Mol. Res. 3 (2), 251-257 (2004).
10. El Tai NO, El Fari M, Mauricio 1, Miles MA, Oskam L, El Safi SH, Presber WH, Schonian G. 2001. Leishmania donovani: intraspecific polymorphisms of Sudanese isolates revealed by PCR-based analyses and DNA sequencing. Exp Parasitol. 97(1), 35-44.
11. Gari-Toussaint, M., Lelievre, A., Marty, P., Le-Fichoux, Y. 1994. Contribution of serological tests to the diagnosis of visceral leishmaniasis in patients infected with the human immunodeficiency virus. Trans. R. Soc. Trop. Med. Hyg. 88(3): 301-2.
12. Harris E, Kropp G, Belli A, Rodriguez B and Agabian N. Single-Step Multiplex PCR Assay for Characterization of New World Leishmania Complexes. J. Clinical. Microbiol. 36(7), 1989-1995 (1998).
13. Herwaldt BL. 1999. Leishmaniasis. Lancet. 354(9185): 1191-9.
14. Lu HG, Zhong L, Guan LR, Qu JQ, Hu XS, Chai JJ, Xu ZB, Wang CT, Chang KP. Separation of Chinese Leishmania isolates into five genotypes by kinetoplast and chromosomal DNA heterogeneity. Am J Trop Med Hyg. 1994 Jun; 50(6):763-70.
15. Maalej 1A, Chenik M, Louzir H, Ben Salah A, Bahloul C, Amri F, Dellagi K. 2003. Comparative evaluation of ELISAs based on ten recombinant or purified Leishmania antigens for the serodiagnosis of mediterrean visceral lesihmaniasis. Am J Trop Med Hyg; 68(3): 312-20.
16. Manson-Bahr PEC. Diagnosis. 1987. In the Leishmaniases in Biology and Medicine, vol. 2, Clinical Aspects and Control. W Peters & R Killick-Kendrick (eds). New York, Academic Press Inc.: p.709-729
17. Martin SK, Thuita-Harum L, Adoyo-Adoyo M, Wasunna KM. 1998. A diagnostic ELISA for visceral leishmaniasis, based on antigen from media conditioned by Leishmania donovani promastigotes. Ann Trop Med Parasitol. 92(5): 571-7.
18. Monroy Ostria & Sanchez-Tezeda G. 2002. Molecular probes and the polymerase chain reaction for detection and typing of Leishmania species in Mexico. Trans R Soc Trop Med Hyg. 96 (Suppl 1), S101-4.
19. Piarroux R, Azaiez R, Lossi A. M, Reynier P, Muscatelli, Gambarelli F, Fontes M, Dumon H and Quilici M. 1993. Isolation and characterization of a repetitive DNA sequence from Leishmania infantum: development of a visceral leishmaniasis polymerase chain reaction. Am J Trop Med Hyg. 49(3):364-9.
20. Pizzuto M, Piazza M, Senese D. 2001. Role of PCR in diagnosis and prognosis of visceral leishmaniasis in patients co-infected with human immunodeficiency virus type-1. J Clin Microbiol.; 39(1), 357-361.
21. Raj VS, Ghosh A, Dole VS, Madhubala R, Myler PJ, Stuart KD. 1999. Serodiagnosis of leishmaniasis with recombinant ORFF antigen. Am J Trop Med Hyg. 61(3): 482-7.
22. Rajasekariah GH, Ryan JR, Hillier SR, Yi LP, Stiteler JM, Cui L, Smithyman AM, Martin SK. 2001. Optimization of an ELISA for the serodiagnosis of visceral leishmaniasis using in vitro derived promastigote antigens. J Immunol Methods. 252(1-2): 105-19.
23. Sassi A, Louzir H, Ben Salah A, Mokni M, Ben Osman A, Dellagi K. 1999. Leishmanin skin test lymphoproliferative responses and cytokine production after symptomatic or asymptomatic Leishmania major infection in Tunisia. Clin Exp Immunol. 116(1): 127-32
24. Schallig HD, Schoone GJ, Kroon CC, Hailu A, Chappuis F, Veeken H. 2001. Development and application of 'simple' diagnostic tools for visceral leishmaniasis. Med Microbiol Immunol (Berl). 190(1-2): 69-71.
25. Schoone GJ, Hailu A, Kroon CC, Nieuwenhuys JL, Schallig HD, Oskam L. 2001. A fast agglutination-screening test (FAST) for the detection of anti-Leishmania antibodies. Trans R Soc Trop Med Hyg. 95(4),400-1.
26. Senaldi G, Xiao-su H, Hoessli D.C, Bordier C. 2001. Serological diagnosis of visceral Leishmaniasis by a dot-enzyme immunoassay for the detection of a Leishmania donovani-related circulating antigen. J Immunol Methods. 193:9-15.
27. Singh S and Sivakumar R. 2003. Recent advances in the diagnosis of leishmaniasis. J. Postgrad. Mend. 49(1): 55-60.
28. Singh S. Gilman-Sachs A, Chang KP, Reed SG. 1995. Diagnostic and prognostic value of K39 recombinant antigen in Indian Leishmaniasis. J Parasitol.81 (6): 1000-3.
29. Singh S, Kumari V, Singh N. 2002. Predicting kala-azar disease manifestations in asymptomatic patients with latent Leishmania donovani infection by detection of antibody against recombinant K39 antigen. Clin Diagn Lab Immunol.9 (3): 568-72.
30. Singh S, Mohapatra DP, Sivakumar R. 2000. Successful replacement of foetal calf serum with human urine for in vitro culture of Leishmania donovani. J Commun Dis. 32(4): 289-94.
31. Singh S. 1999. Diagnostic and Prognostic markers of anti-Kala-azar therapy and vaccination. IN: Proceeding of V Round Table Conference Series. No. 5. Gupta S & Sood OP (Ed). Ranbaxy Science Foundation, New Delhi. Pp 95-114.
32. Sundar S, Rai M.2002. Laboratory diagnosis of visceral leishmaniasis. Clin Diagn Lab Immunol. 9(5): 951-8.
33. WHO expert committee report. Control of the Leishmaniasis.1991. Technical Report Series 793.
34. Williams, J. E. 1995. Leishmania and Trypanosoma. In medical parasitology. A practical approach. Gillespie, S. H., Hawkey. P. M., Eds. London, Oxford University Press.
35. Wortman G, Sweeney C, Houng H-S, Aronson N, Stiteler J, Jackson J, Ockenhouse C. 2001. Rapid diagnosis of Leishmaniasis by fluorogenic polymerase chain reaction. Am J Trop Med Hyg; 65: 583-87.

## Claims

1. An oligonucleotide primer having a sequence selected from SEQ ID NO: 1, SEQ ID No: 2, SEQ ID NO: 3 and SEQ ID NO: 4 for amplification of the kinesin-related gene of *Leishmania* species.

2. A method for detecting and differentiating visceral leishmaniasis (VL) and post kala-azar-dermal leishmaniasis (PKDL) causing strains of *Leishmania donovani* in a sample, the said method comprising the steps of:
a) isolating DNA from a sample;
b) amplifying the target region from the DNA of step (a) using oligonucleotide primers having SEQ ID NO: 1, SEQ ID No: 2, SEQ ID NO: 3 and SEQ ID NO: 4, and heat stable DNA polymerase to obtain amplified fragments;
c) separating the amplified fragments of step (b); and
d) analyzing the fragments of step (c) to detect and differentiate *VL* and *PKDL* causing strains of *Leishmania donovani* based on the banding pattern of the amplified fragments.

3. The method of claim 2, wherein in step (a) the sample is either clinical sample or culture sample.

4. The method of claim 3, wherein the clinical sample is selected from a group consisting of blood, bone marrow aspirate, bone marrow biopsy, splenic aspirate, splenic biopsy, liver aspirate, liver biopsy, lymph node aspirate, lymph node biopsy, skin scrapping, slit biopsy and other tissue materials.

5. The method of claim 2 wherein in step (b) the heat stable DNA polymerase is *Taq polymerase.*

6. The method of claim 2 wherein in step (b) the amplification is done by polymerase chain reaction.

7. The method of claim 2 wherein in step (c) the separation is done preferably by gel electrophoresis.

8. The method of claim 2 wherein in step (d) the detection is by ethidium bromide or other DNA stains.

9. A diagnostic kit for detection and differentiation of VL and PKDL causing strains of the *Leishmania donovani,* comprising oligonucleotide primers identified by SEQ ID NO: 1, SEQ ID NO: 2, SEQ ID NO: 3, and SEQ ID NO: 4, reaction buffer, *Taq polymerase,* DNA marker, a positive control sample, a negative control sample and instruction manual.

## Patentansprüche

1. Oligonukleotidprimer mit einer Sequenz ausgewählt aus SEQ ID NO: 1, SEQ ID NO: 2, SEQ ID NO: 3 und SEQ ID NO: 4 zur Amplifikation des Kinesin-bezogenen Gens von *Leishmania*-Spezies.

2. Verfahren zur Detektion und Differenzierung von viszerale Leishmaniose (VL) und Post-Kala-Azar-dermale Leishmaniose (PKDL) verursachenden *Leishmania donovani-*Sämmen in einer Probe, wobei das Verfahren die Schritte umfasst:
a) Isolieren von DNA aus einer Probe;
b) Amplifizieren der Zielregion der DNA aus Schritt (a) unter Verwendung von Oligonukleotidprimern mit SEQ ID NO: 1, SEQ ID NO: 2, SEQ ID NO: 3 und SEQ ID NO: 4 und hitzebeständiger DNA-Polymerase, um amplifizierte Fragmente zu erhalten;
c) Separieren der amplifizierten Fragmente aus Schritt (b); und
d) Analysieren der Fragmente aus Schritt (c), um ausgehend von dem Bandenmuster der amplifizierten Fragmente VL und PKDL verursachende *Leishmania donovani*-Stämme zu detektieren und zu differenzieren.

3. Verfahren nach Anspruch 2, wobei die Probe in Schritt (a) entweder eine klinische Probe oder eine Kulturprobe ist.

4. Verfahren nach Anspruch 3, wobei die klinische Probe ausgewählt ist aus einer Gruppe bestehend aus Blut, Knochenmarkaspirat, Knochenmarkbiopsie, Milzaspirat, Milzbiopsie, Leberaspirat, Leberbiopsie, Lymphknotenaspirat, Lymphknotenbiopsie, Hautgeschabsel, Schnittbiopsie und anderen Gewebematerialien.

5. Verfahren nach Anspruch 2, wobei die hitzebeständige DNA-Polymerase in Schritt (b) *Taq*-Polymerase ist.

6. Verfahren nach Anspruch 2, wobei die Amplifikation in Schritt (b) mittels einer Polymerasekettenreaktion durchgeführt wird.

7. Verfahren nach Anspruch 2, wobei die Separation in Schritt (c) bevorzugt mittels einer Gelektrophorese durchgeführt wird.

8. Verfahren nach Anspruch 2, wobei die Detektion in Schritt (d) mit Ethidiumbromid oder anderen DNA-Färbemitteln durchgeführt wird.

9. Diagnostischer Kit zur Detektion und Differenzierung von VL und PKDL verursachenden *Leishmania donovani*-Stämmen, umfassend Oligonukleotidprimer identifiziert durch SEQ ID NO: 1, SEQ ID NO: 2, SEQ ID NO: 3 und SEQ ID NO: 4, Reaktionspuffer, *Taq*-Polymerase, DNA-Marker, Proben zur Positiv- und Negativkontrolle und eine Gebrauchsanweisung.

## Revendications

1. Amorce d'oligonucléotides ayant une séquence choisie parmi SEQ ID NO: 1, SEQ ID NO: 2, SEQ ID NO: 3 et SEQ ID NO: 4 pour l'amplification du gène de l'espèce *Leishmania* lié à la kinésine.

2. Procédé de dépistage et de différenciation de la leishmaniose viscérale (LV) et de la leishmaniose dermique post kala-azar (LDPK) à l'origine de souches de *Leishmania donovani* dans un échantillon, ledit procédé comprenant les étapes consistant à :
a) isoler l'ADN à partir d'un échantillon ;
b) amplifier la région cible à partir de l'ADN de l'étape (a) en utilisant les amorces d'oligonucléotides ayant SEQ ID NO: 1, SEQ ID NO: 2, SEQ ID NO: 3 et SEQ ID NO: 4, et l'ADN-polymérase thermostable pour obtenir des fragments amplifiés ;
c) séparer les fragments amplifiés de l'étape (b) ; et
d) analyser les fragments de l'étape (c) pour détecter et différencier la *LV* et la *LDPK* à l'origine de souches de *Leishmania donovani* sur la base du motif de bandes des fragments amplifiés.

3. Procédé selon la revendication 2, dans lequel à l'étape (a) l'échantillon est soit un échantillon clinique, soit un échantillon de culture.

4. Procédé selon la revendication 3, dans lequel l'échantillon clinique est choisi parmi le groupe constitué par du sang, un prélèvement de moelle osseuse, une biopsie de moelle osseuse, un prélèvement splénique, une biopsie splénique, un prélèvement hépatique, une biopsie hépatique, un prélèvement de ganglion lymphatique, une biopsie de ganglion lymphatique, un prélèvement cutané, une biopsie par éraflure et d'autres matières tissulaires.

5. Procédé selon la revendication 2, dans lequel à l'étape (b) l'ADN-polymérase thermostable est une *polymérase Taq.*

6. Procédé selon la revendication 2, dans lequel à l'étape (b) l'amplification est effectuée par réaction de polymérisation en chaîne.

7. Procédé selon la revendication 2, dans lequel à l'étape (c) la séparation est de préférence effectuée par électrophorèse sur gel.

8. Procédé selon la revendication 2, dans lequel à l'étape (d) le dépistage s'effectue avec du bromure d'éthidium ou d'autres marqueurs de l'ADN.

9. Trousse de diagnostic pour le dépistage et la différenciation de la *LV* et de la *LDPK* à l'origine de souches de *Leishmania donovani,* comprenant des amorces d'oligonucléotides identifiées par SEQ ID NO: 1, SEQ ID NO: 2, SEQ ID NO: 3 et SEQ ID NO: 4, un tampon de réaction, la *polymérase Taq,* un marqueur d'ADN, un échantillon de contrôle positif, un échantillon de contrôle négatif et un manuel d'utilisation.


### SEQUENCE LISTING

<110> All India Institute of medical Science
<120> Oligonucleotides for detection of Leishmaniasis and methods thereof,
<130> PCT002
<140> 1598/DEL/03
   <141> 2003-12-23
<160> 4
<170> PatentIn version 3.1
<210> 1
   <211> 17
   <212> DNA
   <213> Artificial Sequence
<220>
   <223> Primer was synthesised in lab
<400> 1
   ctagagcagc agcttcg 17
<210> 2
   <211> 17
   <212> DNA
   <213> Artificial Sequence
<220>
   <223> Primer was synthesised in the lab
<400> 2
   cttgagcagc agcttcg 17
<210> 3
   <211> 17
   <212> DNA
   <213> Artificial Sequence
<220>
   <223> Primer was synthesised in lab
<400> 3
   cgtggccctc gtgttct 17
<210> 4
   <211> 17
   <212> DNA
   <213> Artificial Sequence
<220>
   <223> Primer was synthesised in Lab for use
<400> 4
   cgcggccctc gtgtcct 17